# EUROPEAN PATENT SPECIFICATION

(11) **EP 2 918 236 B1**
(45) Date of publication and mention of the grant of the patent: **10.10.2018**
(21) Application number: 14160126.0
(22) Date of filing: 14.03.2014
(51) Int. Cl.: A61B 17/70

(54) **Device for placing a receiving part onto a head of a bone anchoring element**
Vorrichtung zur Platzierung eines Aufnahmeteils auf dem Kopf eines Knochenverankerungselements
Dispositif pour la mise en place d'une partie de réception sur une tête d'un élément d'ancrage osseux

(43) Date of publication of application: 16.09.2015
(73) Proprietor: Biedermann Technologies GmbH & Co. KG, 78166 Donaueschingen (DE)
(72) Inventor: Biedermann, Lutz, 78048 VS-Villingen (DE); Matthis, Wilfried, 79367 Weisweil (DE); Dandanopoulos, Dimosthenis, 78056 VS-Schwenningen (DE); Biedermann, Timo, 78647 Trossingen (DE)
(74) Representative: Prüfer & Partner mbB Patentanwälte · Rechtsanwälte

(56) References cited:
- WO-A1-2013/187928
- US-A1- 2007 043 378
- US-A1- 2008 200 918
- US-A1- 2010 114 108
- US-A1- 2013 018 428
- US-A1- 2013 023 941
- US-A1- 2013 144 346
- US-B1- 6 440 133

## Description

The present invention relates to a device for placing a receiving part of a bone anchoring device onto a head of a bone anchoring element. The present invention further relates to such a receiving part of a bone anchoring device. The device comprises a holding portion, a collet for engaging the receiving part and a corresponding engagement feature.

Fractures of the spinal column, arthrodesis, scoliosis, etc. are often treated by stabilizing the spinal column using bone anchors applied to two or more vertebrae and connecting these via rigid or dynamically flexible stabilization rods such as to define a predetermined relative position and movement tolerance for each of the vertebrae involved. In operation, vertebrae are first selectively supplied with bone anchors and a stabilization rod is then attached to connect each of the bone anchors. The connection is established by means of a receiving part, which connects to a head of the bone anchoring element and receives a spinal rod in a rod receiving channel thereof.

In recent years, there has been a tendency to assemble such bone anchoring devices in situ. Methods of in situ assembling of the bone anchoring devices advantageously allow - since the receiving parts are attached to the bone anchors in a subsequent step - visual inspection of several structures in place such as particularly neurological structures etc., which may be critical. New clinical methods and surgical steps also become possible. Moreover, particular advantages arise in that a preparation of very small MIS (minimally invasive surgery) accesses is made possible.

For example a bone anchoring element is anchored in a vertebra first, whereafter a receiving part is placed onto the head of the bone anchoring element. However, such construction necessitates that the receiving part allows a function to load the head from the bottom. As a consequence, the bottom opening has an increased diameter and the head has to be received by a portion of pressure element or inner cap provided within the receiving part, which allows encompassing the head and locking against a seat formed adjacent the bottom opening. One early example is described in document US 6,471,705 A, or EP 1 105 057 A1, by applicant.

In view of the above, a need has arisen to also provide a device suitable for placing a receiving part onto a head of a bone anchoring element.

Document US 2011/0166606 A1 discloses a reduction tool designed to engage a body portion having a rod receiving channel and to insert a spinal rod therein. The tool comprises an outer tube having engagement features at the distal end and two opposite slots through which the rod is inserted, and an inner tube which advances a locking cap and the rod. At the proximal end, the outer tube also has two opposite slots such that a pivot point is formed in a middle portion wherein a compression of the proximal end of the outer tube results in an expansion of the distal end. As a consequence of such manipulation, engagement of the receiving part by means of protruding engagement features provided at the distal end of the outer tube can be facilitated. Upon engagement, those protrusions are led into corresponding recesses formed in an outer circumferential surface of the receiving part.

However, the above described reduction tool is configured to engage a receiving part in a state preassembled with the bone anchoring element, and further, due to the pivoting expansion to engage and disengage the receiving part at its circumferential surface, much space is need in a patient's body rendering the tool less suitable for minimal invasive surgery and in situ operation.

Document WO 2013/187928 A1 discloses a pedicle screw extension assembly for use in percutaneous spinal fixation. The assembly has an outer screw extension and a hollow elongate inner sleeve. At a distal end of the inner sleeve, radially extending flanges are provided to form anchor engaging members. The flanges may engage with undercuts provided adjacent a top end of a yoke. The extension is secured to the yoke of the bone anchor by an axial movement rather than rotation. The outer screw extension has a cam element and an inclined surface such as to bias the arms of the inner sleeve having the flanges at the distal ends radially inwards, and to release the same when the extension is secured to the yoke. The connection is this state secured by mutually engaging lips of the inner sleeve and the outer screw extension.

Document US 6,440,133 B1 discloses a rod reducer instrument, w herein a fastener engaging member is provided having flexible arms with an enlarged distal portion forming inwardly facing prongs to be secured to a yoke, and a rod channel. A reducing member can be axially displaced in a manner of a collet chuck to secure the prongs in recesses formed in an outer contour of the yoke.

Document US 2009/0163963 A1 discloses a driving apparatus for implanting spinal rod fixation devices. Such a fixation device includes an anchor member and a coupling member. The driving apparatus immobilizes both members relative to each other and to the driving apparatus itself during insertion of the anchor member. The driving apparatus further includes a driver member engageable with the anchor member thereby closely fitting between upstanding walls of the coupling member to prevent rotation thereof. The driving apparatus also includes a locking member comprising an outer body and an inner actuating rod. At a distal end of the body there are provided two flexible arms formed with lugs configured to be deflected into ports in a bore of the coupling member to lock the connection when the actuating rod is advanced to spread the arms of the body.

Document US 2012/0296171 A1 discloses an inserter including a driver and a receiver member wherein a head of a bone anchor to be inserted is securely connected to the inserter by virtue of the receiver member which is used in a collet-like fashion. The receiver member thereby receives the head and a cylindrical body is slid over a conical portion of the receiver member to compress deflectable flanges onto the head. A driving operation may then be performed with regard to the bone anchor. In this embodiment, however, the receiver member is part of the inserter and cannot be released and used to receive a rod.

It is thus an object to provide a device for placing a receiving part onto a head of a bone anchoring element, which improves known methods and devices.

The object is solved by a device for placing a receiving part of a bone anchoring device onto a head of a bone anchoring element according to claim 1. The object is also solved by a receiving part for connecting a bone anchoring element having a head and a shank to a spinal rod according to claim 15. Advantageous aspects and embodiments become apparent from the dependent claims.

The device includes a holding device and a collet held by the holding device. The collet has a distal end portion which is configured to be flexibly expanded and/or compressed, which allows engaging and/or disengaging a corresponding receiving part. An engagement feature is provided at an outer contour, or outer surface, of the distal end portion of the collet. The engagement feature may engage a portion of an inner wall formed in a rod receiving channel or in a bore of a receiving part.

More specifically, the engagement feature is not provided at an inner surface of a tube or shaft as proposed in prior art, but on its outer surface such as to engage an inner wall of the receiving part. The inner wall may be that of the inner bore extending for example from a top end to the bottom end of the receiving part, or may be that of the rod receiving channel which is typically formed extending from the top end of the receiving part. Other inner walls are possible as well as long as engagement takes place within a perimeter of the receiving part and the release operation is not directed into a space beyond such perimeter, in particular by expansion. In embodiments described herein, the device performs compression at its distal end when releasing the receiving in place.

Consequently, an advantage arises in that it is not necessary to engage the receiving part on any of its circumferential outer surfaces. This in turn allows to create narrower channels through the tissue of the human body to approach the bone under concern. Hence, aspects and embodiments of the invention are particularly applicable in minimally invasive surgery (MIS) and in situ assembly.

Engagement and/or disengagement is facilitated by radial expansion or compression of the distal end portion of the collet, respectively. It is not necessary to use the inner thread which is generally available at inner walls of receiving parts to facilitate locking by means of a locking screw. Instead, quick locking and releasing of the receiving part with an improved tactile response becomes possible when placing the receiving part onto the head.

With regard to the receiving part as proposed herein, only minor structural changes have to be applied, if any. For example, engagement features may be provided at the inner walls of the rod receiving channels. These may be protrusions, spring-like projections, noses, recesses, and similar means which allow a latching connection with corresponding features of the device. Such engagement features may even be provided within a region of an inner thread - if present. According to specific embodiments, even an inner thread might be considered to provide corresponding latching features into which the engagement features of the device expand during engagement.

A particularly advantageous embodiment becomes apparent in conjunction with an application to a bone anchoring device, wherein an inner cap is provided within the receiving part, the inner cap clamping the head of the bone anchoring element thereby allowing a frictionally limited movement of the clamped bone anchoring element, when the device has not yet been finally locked. In this situation, the device allows a particularly efficient placement method for the receiving part onto the head.

Advantageous features and aspects will become more apparent from a detailed description of a specific embodiment taken in conjunction with the accompanying drawings, wherein:
- Fig. 1: shows a perspective view of the device according to an embodiment of the invention with the collet in a locked position;
- Fig. 2: same as Fig. 1, but in use just prior to a placement of the receiving part onto the head and with the operation portion being removed;
- Fig. 3: same as Fig. 1, but in a disassembled state;
- Fig. 4A: shows a cross section of the holding portion of the device shown in Fig. 1;
- Fig. 4B: same as Fig. 4A, but in an perspective view from a top;
- Fig. 4C: same as Fig. 4B, but seen from a bottom perspective;
- Fig. 5A: shows a cross section of a tube of the collet of the device shown in Fig. 3;
- Fig. 5B: shows an enlarged view of a proximal end of the tube shown in Fig. 5A;
- Fig. 5C: shows an enlarged view of the distal end portion of the collet;
- Fig. 6A: shows a perspective view of a proximal end portion of the collet according to the embodiment shown as in Fig. 3;
- Fig. 6B: same as Fig. 6A, but from a bottom perspective;
- Fig. 6C: same as Fig. 6A, but in a cross-sectional view;
- Fig. 7A: shows a perspective view of a plunger of the device shown in Fig. 3;
- Fig. 7B: shows an enlarged view of a tip portion of the plunger shown in Fig. 7A;
- Fig. 8: shows a fixation device of the device shown in Fig. 1, formed as a follower pin;
- Fig. 9: shows an operation portion of the device shown in Fig. 1;
- Fig. 10A: shows a cross-sectional view of a receiving part according to an embodiment of the invention, along with a top view thereof;
- Fig. 10B: same as Fig. 10A, but in a perspective view;
- Fig. 11A: shows a cross-sectional view of an inner cap according to an embodiment of the invention along with a top view thereof;
- Fig. 11B: same as Fig. 11A, but in a perspective view;
- Fig. 12: shows in a cross-sectional view a first stage of using the device according to the invention as shown in Fig. 1;
- Fig. 13A: shows in an enlarged perspective view the distal end portion of the collet and the receiving part in the same state as shown in Fig. 12;
- Fig. 13B: same as Fig. 13A, but in a second stage of advancing the distal end portion into the receiving part;
- Fig. 13C: same as Fig. 13B, but in a third stage wherein the distal end portion is engaged with inner walls of the receiving part;
- Fig. 14: shows a cross-sectional view of the device in the same state as shown in Fig. 13C with the distal end portion of the collet being locked by the plunger;
- Fig. 15A: shows in a partially cross-sectional view a fourth stage of using the device wherein the device is guided to a head of a bone anchoring element in the locked state as shown in Fig. 14;
- Fig. 15B: same as Fig. 15A, but in a fifth stage with the head introduced and snapped into the inner cap;
- Fig. 15C: same as Fig. 15B, but in a sixth stage with the device drawn away from the receiving part;
- Fig. 16A: same as Fig. 15C, but in a seventh stage with the distal end portion being unlocked;
- Fig. 16B: same as Fig. 16A, but showing the proximal portion of the device with the tensioned resilient member;
- Fig. 16C: shows a ninth stage of a method of using the device, wherein the unlocked distal end portion of the collet is released from the receiving part.

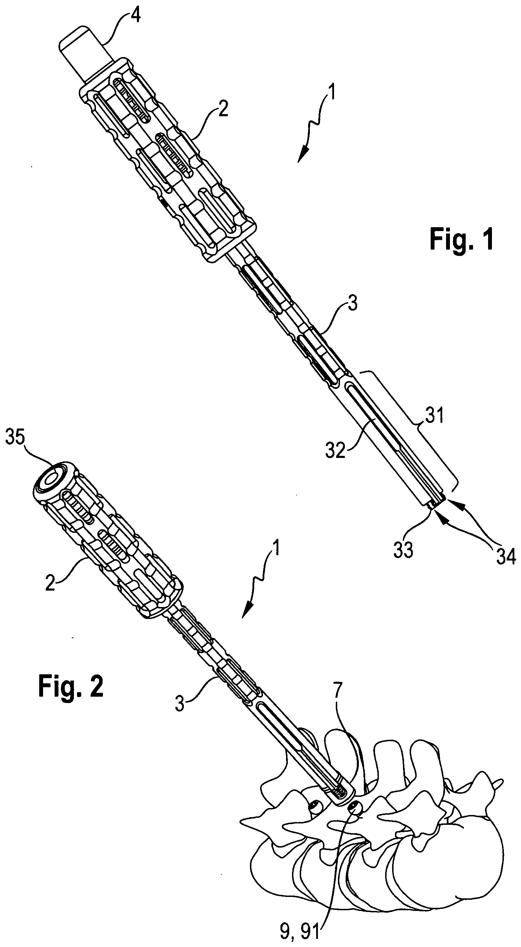

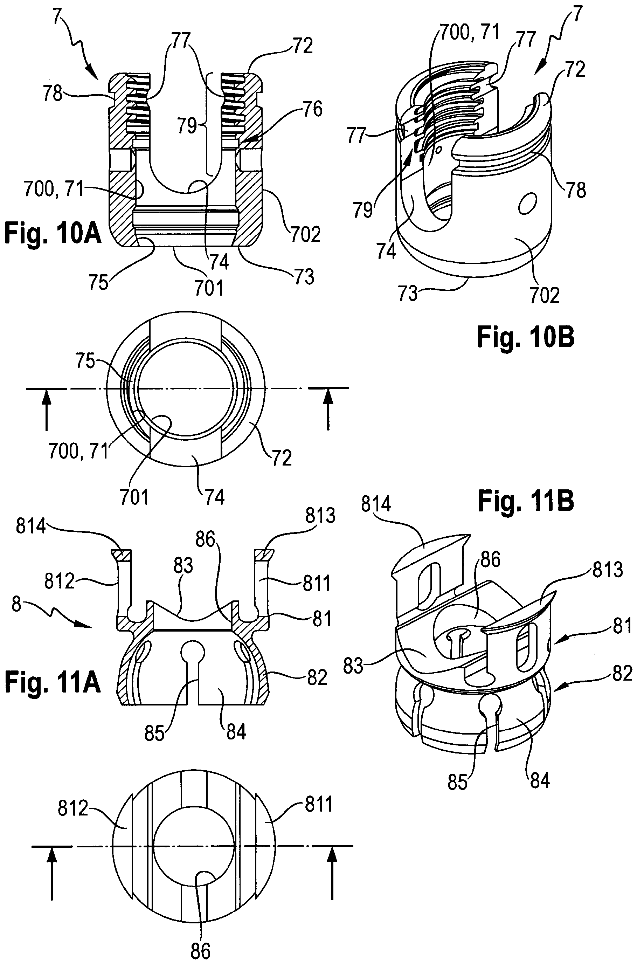

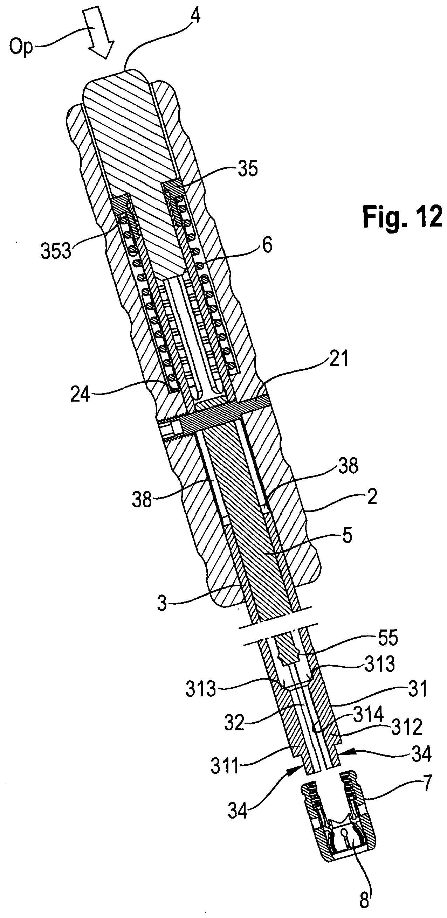

In the following, an embodiment of the device is described with respect to Figs. 1-9, a corresponding embodiment of a receiving part with an inner cap is described with reference to Figs. 10A-11B, and an illustrative example of a method of using the device and receiving part of both embodiments is given with reference to Figs. 12-16C.

An overview of the device 1 for placing a receiving part onto a head of a bone anchoring element is given in Figs. 1-3. The device 1 according to this embodiment comprises a holding portion 2, a collet 3, an operation portion 4, a plunger 5 and a resilient member 6. A fixation device 21 provided as a follower pin is supplied to the holding portion to establish a connection with plunger 5, and a cap-like proximal end portion 35 is provided to the collet 3 to allow attaching the operation portion 4 to a proximal end of the collet 3, and to provide a proximal abutment face 353 for the resilient member 6 as will be described below.

Fig. 1 shows the device 1 in a state, wherein the operation portion 4 is not pressed, as a consequence of which the collet 3, or its distal end portion 31, respectively, is in a locked state, since a tip portion 52 of plunger 5 extends into the distal end portion 31 as will be explained in detail below.

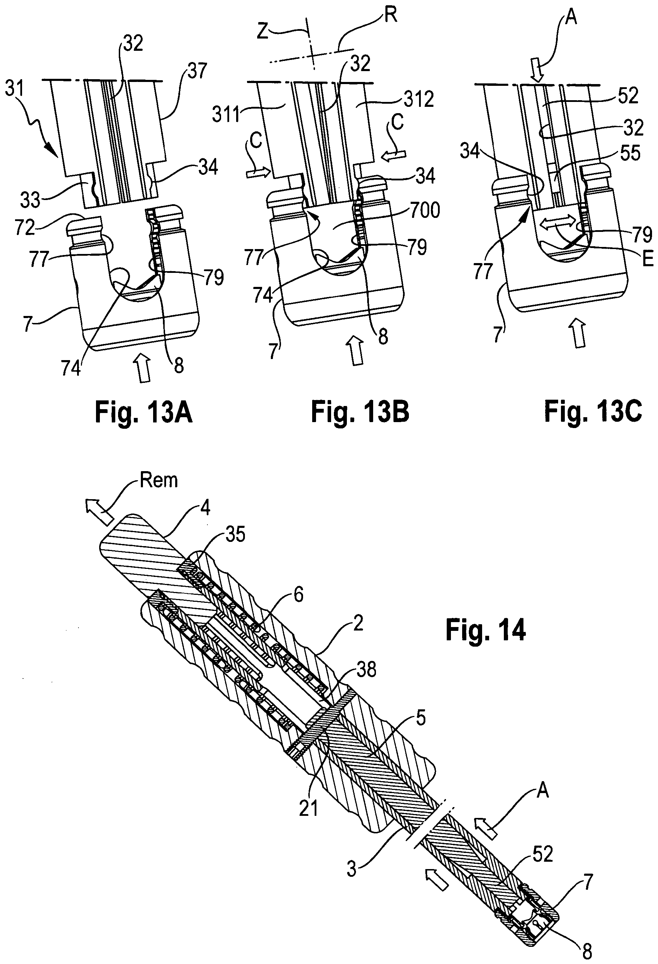

Similarly, the device 1 as shown during use in Fig. 2 also is in a locked state, wherein, however, the operation portion 4 has been removed from the device 1. In a locked state, the receiving part 7 securely adheres to the distal end portion of the collet 3 and may thus be safely guided through an incision channel cut into the tissue (not shown) towards a head 91 of a bone anchoring element, which has already been anchored in a pedicle of a vertebra shown in Fig. 2. The state shown in Fig. 2 substantially corresponds to that shown in Fig. 14 illustrating the method of use, which will be discussed in detail below.

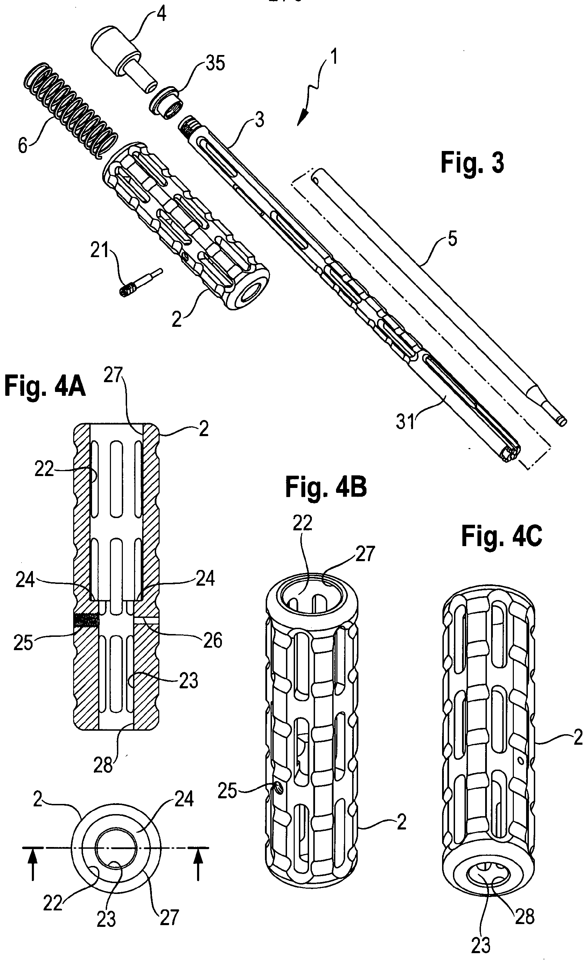

In what follows, an explanation of the constituent parts of the device 1 is given first with respect to Figs. 4A through 9.

The holding portion 2 is shown in Figs. 4A-4C in cross-sectional, top and perspective views, respectively. The holding portion 2 of this embodiment is of a substantially tubular shape with a textured profile at its outer surface to allow safe gripping by an operating staff. In use, the holding portion 2 is held with one hand, wherein a thumb may for example be used to press the operation portion 4. The holding portion 2 includes an inner bore extending from a proximal end 27 towards a distal end 28. A first bore portion 22 having a comparatively large diameter extends from the proximal end portion 27 and a second bore portion 23 extends from the distal end portion 28. The second bore portion 23 has a smaller diameter as compared with the first bore portion 22. At the transition between the first bore portion 22 and the second bore portion 23 which both extend along a common longitudinal axis, a shoulder 24 serving as a distal abutment face for one end of the resilient member 6 described below is formed.

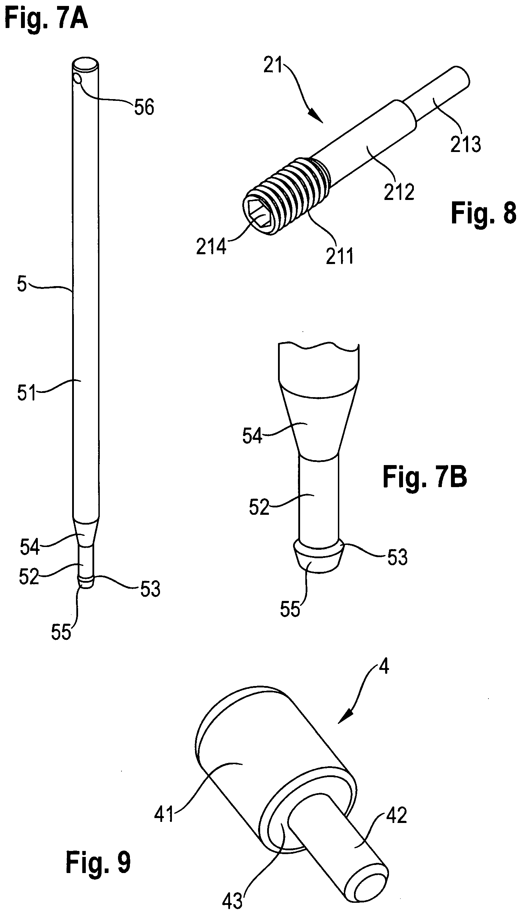

A threaded bore 25 transversely extends through a wall of the holding portion 2, and a further bore having a smaller diameter extends through the wall on an opposite side along the same axis as threaded bore 25. Bores 25, 26 are configured to receive fixation device 21 as shown in Fig. 8. More specifically, fixation device 21 has a threaded section 211 configured to be threaded into the threaded bore 25, a narrower unthreaded pin portion 213 configured to fit into the bore 26 of the holding portion 2, and a cylindrical middle portion 212 configured to slidably fit through an oblong aperture 38 of collet 3 described below.

The fixation device 21 functions as a follower pin and fixes the plunger 5 to the holding portion 2 such that both portions 2 and 5 together form a first unit of the device 1.

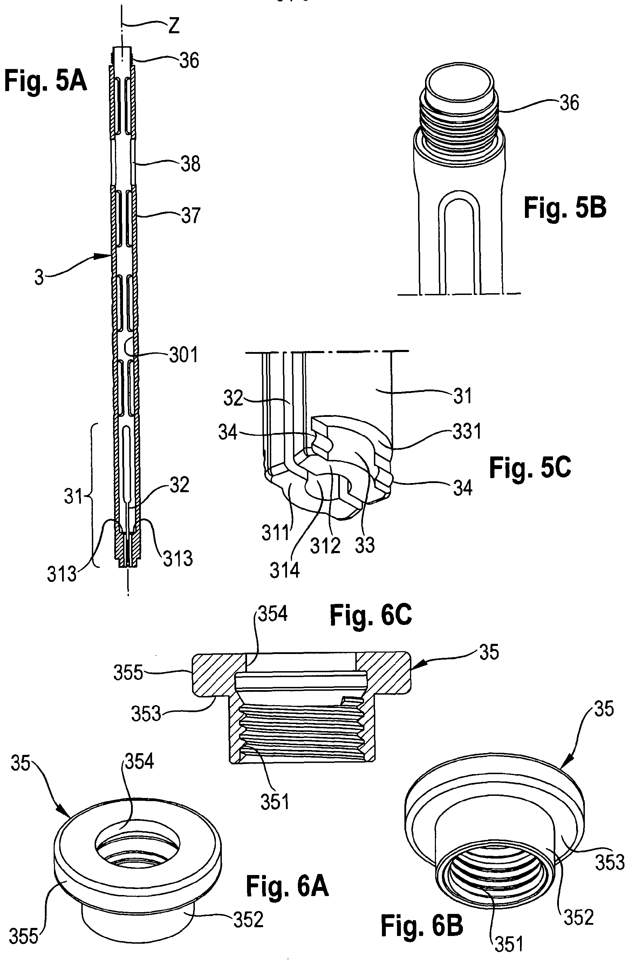

The collet 3 is depicted in Figs. 5A and 5B with its proximal end portion 35 formed as a separate cap-like member depicted in Figs. 6A-6C. The collet 3 is of substantially tubular shape (tube 37) and its outer diameter is selected such as to slidably fit into the narrower second bore portion 23 of holding portion 2. When being assembled, its longitudinal axis Z is identical with that of the first and second bore portions 22, 23 of holding portion 2.

The collet 3 has a distal end portion 31 which is defined by two tongues 311, 312 which in turn are formed by two opposite slots 32 extending from a tip portion along the longitudinal axis Z up to a predetermined distance from the tip. The tip portion 33 of the distal end portion 31 is recessed from the overall cylindrical shape of the collet 3 and shaped such as to fit into the rod receiving channel and inner bore of the receiving part 7. Upon engagement of the distal end portion 31 with the receiving part 7 an abutment face 331 of the collet 3 abuts on a top face of the receiving part 7. Moreover, each two rounded projections are formed as engagement features 34 on each of the tongues 311, 312 within the tip portion 33 of the collet 3. These projections extend in a lateral or radial direction R from the collet 3.

The length of the slots 32 and the thickness of the walls of the collet 3 are dimensioned and the material selected such that both tongues 311, 312 are provided with resiliency such as to be expanded or compressed depending on forces exerted during operation of the device 1.

For example, in this embodiment, the plunger 5 as shown in Fig. 7A and 7B is used to prevent compression and even to expand the tongues 311 and 312 of collet 3. Plunger 5 is substantially rod-shaped and has a cylindrical portion 51 having a diameter configured to slidably fit into an inner bore 301 of collet 3. Plunger 5 further has a partially cylindrical tip portion 52 having a smaller diameter as compared with the cylindrical portion 51 and a conical portion 54 at a transition between the cylindrical portion 51 and the tip portion 52. The diameter of the cylindrical part of tip portion 52 of plunger 5 is substantially the same as a diameter of a narrow diameter bore hole 314, which is provided to extend from the shoulder 313 forming an end of inner bore 301 of collet 3 up to the tip of the tip portion 33.

In this specific embodiment, tip portion 52 further includes a conical tip portion 55 having a slightly increasing diameter larger than that of the cylindrical part of the tip portion 52.

Since the plunger 5 is configured to slide within the inner bore 301 of collet 3 the tip portion 52 of plunger 5 is allowed to enter the narrow diameter bore 314 of the collet 3 wherein by virtue of the conical tip portion 55 having the larger diameter, the tongues 311, 312 are even radially expanded in this embodiment. The length of the tip portion 52 including the conical portion 55 substantially corresponds to the length of the narrow diameter bore 314. From the above, it becomes clear that once the tip portion 52 has entered the bore 314 of collet 3, compression of the tongues 311, 312 is impeded and even a slight expansion is facilitated.

As explained above, the holding portion 2 and the plunger 5 form a common unit in this embodiment. On the other side, collet 3 is allowed to move with respect to that unit along the longitudinal axis Z. The extent of such movement is defined by the length of oblong hole 38 through which the fixation device 21 extends as a follower pin in the assembled state. Figs. 12 and 14 show respective states in cross-sectional illustrations. More specifically, Fig. 12 shows a state in which the holding portion 2 and plunger 5 are moved towards a proximal direction and the collet 3 is moved towards a distal direction, which yields a state in which a tip portion 52 and conical tip portion 55 of plunger 5 are retracted from narrow diameter hole 314 and shoulders 313. In this state, tongues 311, 312 may be compressed or expanded thereby representing an unlocked position ("second release position"). In this unlocked or release position, the fixation device 21 is positioned at a proximal end of oblong hole 38 of collet 3. The unlocked or release position allows an easy attachment or removal of the tongues 311, 312 to the receiving part 7.

On the contrary, Fig. 14 shows a second state, in which the collet has been retracted towards the proximal direction and the holding portion 2 and plunger 5 are uniformly moved forward. In this state, tip portion 52 enters the narrow diameter bore 314 of collet 3 thereby preventing compression of the tongues 311, 312. This state corresponds to a lock position ("first lock position") of the plunger 5 with respect to collet 3. The first lock position may advantageously achieved, for example when the projections or engagement features 34 are engaged into respective features provided at the receiving part 7. Hence, a safe and secure engagement of the receiving part 7 may be accomplished. As can be seen from Fig. 14, the fixation device 21 is thereby positioned at the opposite distal end of oblong hole 38. Oblong hole 38 limits the movement of plunger 5 with respect to collet 3, and its ends toward the proximal direction and distal direction define, for example, the first lock position and the second release position, respectively.

Returning to the description of the collet 3, a proximal end portion 35 is achieved by a separate cap-like member shown in Figs. 6A through 6C. Proximal end portion 35 includes a cylindrical tubular portion 352 comprising an inner thread 351, that may be threaded upon an outer thread 36 provided at an end of collet 3 as shown in Fig. 5B. Proximal end portion 35 further has an inner bore 354 sized to receive therein a pin-like projection 42 of operation portion 4 shown in Fig. 9. The cylindrical portion 352 has the same outer diameter as that of the cylindrical tube portion 37 of collet 3. Further, a cap-like annular projection 355 is provided whose diameter corresponds to that first bore portion 22 of holding portion 2. As a consequence, a surface is formed on its bottom side, which serves as a second abutment face 353 for a second proximal end of resilient member 6.

As can be seen from Figs. 12 or 14, the resilient member 6 is held within the first bore portion 22 of holding device 2 and urges the proximal end portion 35 of collet 3 away from abutment face 24 of holding portion 2 into the proximal direction. Thus, in a state where no forces are exerted onto operation portion 4, as shown in Fig. 14, the holding portion 2 and plunger 5 are moved towards a distal direction, i.e., towards the receiving part when being engaged, and the collet 3 is retracted towards the proximal direction together with its proximal end portion 35 and operation portion 4 which thus serve together as a second unit of the device 1.

Next, an embodiment of the receiving part 7 suitable for interaction with device 1 is described with reference to Figs. 10A, 10B. The receiving part 7 includes a bore 700 extending from a first top end 72 towards a second bottom end 73 wherein an opening 701 is formed at the bottom end 73. Adjacent to the opening 701, a seat 75 is formed which defines an accommodation space for accommodating a head receiving portion 82 of an inner cap 8 configured to clamp a head 91 of a bone anchoring element 91 such as that shown in Fig. 2. The bore 700 includes an inner wall 71, wherein at an upper portion of the inner wall 71, an inner thread for receiving a locking device (not shown) such as a setscrew is formed. An abutment feature 76 such as a recess is formed in a middle portion of the inner wall 71 which is designed to receive corresponding abutment features 813, 814 of the inner cap 8 shown in Fig. 11A and 11B.

Receiving part 7 also includes a rod receiving channel 74 which extends from the first top end 72 and has substantially a U-shape. The rod receiving channel 74 thus forms two legs and two inner walls 79 having the U-shape. As can be seen particularly from Fig. 10B, each two recesses or engagement features 77 having rounded edges are formed in an upper portion of the inner walls 77. These engagement features 77 are configured and shaped to receive the engagement features 34 provided at the distal end portion 31 of collet 3 of device 1. The recesses of engagement features 77 similarly extend in a lateral direction perpendicularly to a longitudinal axis of the bore 700, which longitudinal axis is also coincident with the longitudinal axis Z of collet 3 in the assembled state. As a consequence of the lateral, or radial orientation expansion or compression of the distal end portion 31, or of the tongues 311, 312, respectively, facilitates engagement or disengagement.

It may be noted, that the receiving part 7 shown in Figs. 10A and 10B further comprises a classical engagement feature 78 provided at a circumferential surface 702 of the receiving part 7. That engagement feature 78 allows engaging the receiving part 7 externally with another instrument as known in the art.

An example of an inner cap 8 as part of the receiving part is illustrated in Figs. 11A and 11B. The inner cap 8 includes a more or less cylindrical portion 81 and a substantially semi-spherical head receiving portion 82. The head receiving portion 82 has resilient flanges 84 separated by slots 85, which together are configured to receive and clamp the head 91 of bone anchoring element 9 in a snap-on fashion. In the clamped state, a resilient force is still exerted onto the head 91 such as to provide friction to any pivoting movement of the bone anchoring element 9. Such feature provides an advantageous degree of pre-locking for the bone anchoring device.

The cylindrical portion 81 has a diameter substantially corresponding to the diameter of the bore 700 and, as explained above, abutment features 813, 814 provided at the tips of legs 811, 812 may abut against edges of recess 76 to prevent falling off of the inner cap 8 from the bore 700. Moreover, the recess 76 is provided at a distance from the opening 701 of receiving part 7, such that upon introduction of the head 9 through opening 701, the inner cap 8 may move upward allowing the flanges 84 to expand and to snap on the head 9. Upon final locking of the head 9, the inner cap 8 is urged toward the seat 75 such as to compress the flanges 84 onto the spherical head 91 of bone anchoring element 9.

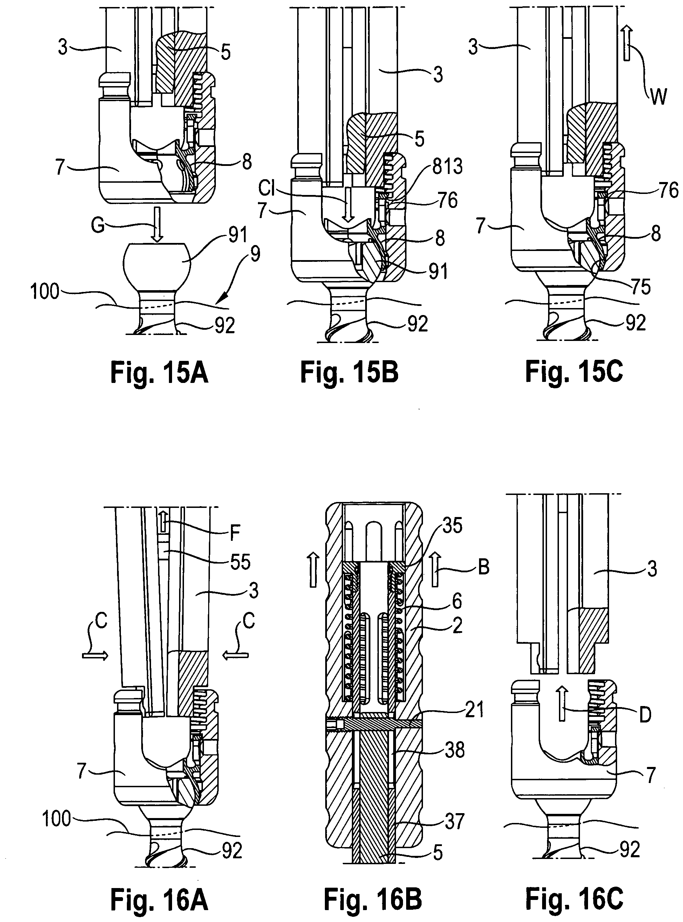

Next, operation of device 1 and receiving part 7 is explained with reference to Figs. 12 through 16C. Therein, Figs. 12 through 14 reveal steps of engaging the receiving part 7, while Figs. 15A through 16C reveal steps placing and releasing the receiving part 7 on the head 91 of the bone anchoring element 9, respectively.

Figs. 12 and 13A show a first stage of the process before engagement of the receiving part 7. As can be seen in Fig. 12, the operation portion 4 is already actuated (arrow Op) against the urging force of the coil spring of resilient member 6. Consequently, the unit comprising operation portion 4 and the collet 3 with proximal end portion 35 is advanced towards a distal direction, or expressed reversely, the unit comprising the holding portion 2 and plunger 5 is retracted towards a proximal direction. As a result, the tip portion 52 including the conical tip portion 55 of plunger 5 is retracted from the narrow diameter hole 314 inside the distal end portion 31 of collet 3. Tongues 311, 312 are then prevented from being resiliently compressed.

A second stage of the process is shown in Fig. 13B. The tip portion 33 of distal end portion 31 is introduced into an inner space of the receiving part 7 formed by bore 700 and rod receiving channel 74. Introduction is accomplished along the longitudinal axis Z of collet 3. As the cross-sectional profile of the tip portion 33 of collet 3 corresponds to the cross section of the inner space of receiving part 7, the tip portion 33 initially may snugly fit into the inner space. However, the engagement features 34 protrude laterally outward from the tip portion 33 and eventually abut on top edges of the rod receiving channel 74. Due to the rounded surface of engagement features 34, tongues 311, 312 deflect radially inward along direction R perpendicular to the longitudinal axis Z, i.e., are compressed towards each other (see arrow C).

As shown in Fig. 13C, the engagement features 34 latch into respective recesses of engagement features 77 thereby allowing the tongues 311, 312 to expand again (see arrow E). Fig. 14 shows the same third stage of the process as shown in Fig. 13C, and it becomes apparent that releasing of the operation portion 4 leads to a retraction of the collet 3, or an advancement of holding portion 2 with plunger 5, see arrow A, due to the urging force of resilient member 6. Thereby, the conical tip portion 55 and the tip portion 52 of plunger 5 enters the narrow diameter hole 314 of collet 3 thereby locking the engagement between collet 3 and receiving part 7 ("first lock position"). More specifically, the tip portion 52 prevents any further compression such that engagement features 34 cannot be released from engagement features 77. Moreover, further tightening is achieved by the conical tip portion 55 which further expands the tip portion 33 and engagement features 34 into engagement features 77 of receiving part 7.

In this situation, as shown in Fig. 14, the operation portion 4 may be removed from device 1 as indicated by arrow *Rem.* Removal of the operation portion 4 guarantees that no undeliberate release of the receiving part 7 becomes possible during further steps of the method. This becomes possible, because the structure of the device 1 and receiving part 7 allows an automatic release after placement of the receiving part and upon a simple withdrawal movement of device 1 as will be explained in the following.

Fig. 15A shows a fourth stage of the method. The assembly of device 1 with engaged receiving part 7 is guided through an incision (not shown) in the tissue towards head 91 of bone anchoring element 9 whose shank 92 has already been driven into the bone material of vertebra 100, see arrow G.

Fig. 15B shows a fifth stage of the method, wherein the head 91 is introduced through the bottom opening 701 into the accommodation space of bore 700 and clicked into the head receiving portion 82 of inner cap 8, see arrow *Cl.* Thereby, prior to being clicked into the head receiving portion 82 and clamped therein in a pre-lock fashion, the inner cap 8 is moved upwards within bore 700 until projections 813 latch into recesses 76 and abut on their upper edges. This abutment prevents the inner cap 8 from moving further upwards. Having reached this position, flanges 84 of head receiving portion 82 are urged to deflect outwards and receive the head 91 therein in snap-on fashion.

As shown in Fig. 15C, the collet 3 is then withdrawn from the bone anchoring element 9, see arrow W, as a result of which the inner cap 8 moves downward within bore 700 (or reversely: the receiving part moves up) and is pressed against the seat 75 (or reversely: the seat is pressed against the inner cap) with the force of withdrawal. As a consequence, the head 91 is clamped within inner cap 8 and the receiving part 7 cannot further move up and cannot easily be released from the clamped state.

Fig. 16A shows a seventh stage of the method wherein, as shown in Fig. 16B, further withdrawal leads to a compression of resilient member 6, because it is the holding portion 2 that is effectively drawn up by the operating staff and not the collet 3. Once the head 91 and inner cap 8 are in a clamped state as shown in Fig. 15C, the collet 3 cannot be further drawn up and the holding portion 2 is moved with respect to collet 3 under increasing tensioning or biasing force exerted on the coil spring of resilient member 6, see arrow B. This in turn leads to a retraction (see arrow F) of plunger 5 and conical tip portion 55 from the distal end portion 31 of collet 3. This position of plunger 5 corresponds to a released or unlocked position with respect to the distal end portion 31, and further withdrawing the device 1 allows compression of the tongues 311, 312 (see arrow C in Fig. 16A) and therefore release of receiving part 7. As noted above, upon drawing up the holding portion, the resilient member is tensioned with an increasing force. Thereby, the resilient member 6 is configured to be tensioned by an amount of force in a state, in which the plunger 5 releases the distal end portion 31, which amount of force is less than a clamping force exerted by the flexible head receiving portion 82 of the inner cap 8. Hence, it is assured that the receiving part is released before the head unclamps from inner cap 8, even if it would not be pressed against seat 75 and thus locked.

As shown in the eighth stage, revealed in Fig. 16C, the collet 3 may disengage from the receiving part 7 (see arrow D) which is thus safely placed onto the head 91 of bone anchoring element 9. Simultaneously, as forces are not exerted any more onto device 1, the collet 3 within device 1 is immediately retracted towards the proximal direction again under the urging force of resilient member 6 with respect to holding portion 2, such that device 1 returns into its initial locked state.

In the state achieved so far, the receiving part is pre-locked, which means that by virtue of friction created between the head receiving portion 82 and the clamped head 91, the receiving part has given posture with respect to the bone anchoring element 9, which may, however, easily be repositioned by hand or an instrument. Further possible steps then include supplying a spinal rod (not shown) to the receiving part, wherein the spinal rod is inserted into the rod receiving channel 74. Moreover, a locking screw (not shown) can then screwed into the inner thread at the inner wall 79 of the receiving part 7, which locks the spinal rod, which in turn presses onto the V-shaped groove within the cylindrical portion 81 of the inner cap 8 such as to firmly and finally lock the receiving part 7 and head 91.

It may be noted that various modifications may be applied to aspects and embodiments of the invention which may be considered to come within the scope of the appended claims. In the above embodiments, metal materials such as titanium, or nickel titanium alloys or Nitinol or other body-compatible metals are employed for each of the components. However other materials such as plastic materials, or PEEK, etc. may also be used.

Moreover, in the above embodiment, a receiving part employing an inner cap having a specific head clamping function is used. Nevertheless, other structures of receiving parts having pressure elements or other inner caps may also be used.

In the above embodiments, expansion of tongues is used to engage with engagement features provided at a receiving part. However, it is also contemplated that movable engagement parts may be provided at a tip of a shaft which provide an expanding movement within an inner space at a receiving part upon engagement.

## Claims

1. A system of a device (1) for placing a receiving part (7) of a bone anchoring device onto a head (91) of a bone anchoring element (9) and the receiving part (7), comprising:
the receiving part (7); and
the device (1), which further comprises
a holding device (2),
a collet (3) having a longitudinal axis (Z) and being movably held by the holding device (2) and having a tubular shape, wherein the collet (3) includes a distal end portion (31) configured to be flexibly expanded (E) and/or compressed (C) in a radial direction (R) perpendicular to the longitudinal axis (Z),
wherein an engagement feature (34) is provided at an outer contour of the distal end portion (31) of the collet (3) configured to engage an engagement feature (77) of a U-shaped inner wall (79) formed in a rod receiving channel (74) of the receiving part (7),
a plunger (5) configured to selectively lock and release the distal end portion (31) of the collet (3) at the receiving part (7), when the engagement feature (34) engages the engagement feature (77) of the inner wall (79) formed in the receiving part (7), wherein
the plunger (5) has a rod-shape and at least partially extends through the collet (3).

2. The system of claim 1, wherein
the collet (3) is configured to be movable with respect to the plunger (5) in a direction along its longitudinal axis (Z) between at least
a first lock position, in which the distal end portion (31) of the collet (3) is prevented by a portion of the plunger (5) from being compressed, and
a second release position in which the distal end portion (31) may be resiliently compressed (C).

3. The system of claim 2, wherein
the plunger (5) is fixedly coupled to the holding device (2).

4. The system of one of claims 1 to 3, wherein
the plunger (5) is coupled to the holding device (2) via a fixation device (21) extending though an oblong aperture (38) formed in the tubular-shaped collet (3).

5. The system of one of claims 1 to 4, further comprising:
a resilient member (6), which is coupled between the holding device (2) and a proximal end portion (35) provided at the collet (3) so as to urge the collet (3) into a proximal direction in order to establish the first lock position.

6. The system of claim 5, wherein
the plunger (5) has a tip portion (52), which is configured, in the first lock position, to enter the distal end portion (31) of the collet (3) such as to prevent compression (C) thereof.

7. The system of claim 6, wherein
the tip portion (52) of the plunger (5) further comprises a tapered or conical portion (55) configured to further expand the distal end portion (31) of the collet (3) in addition to preventing compression.

8. The system of one of claims 5 to 7, further comprising
an operation portion (4), which is removably connected, fixed to, or integrally formed with the proximal portion (35) provided at the collet (3) configured to allow an operator to move the collet (3) with respect to the holding device (2) and plunger (5) against an urging force of the resilient member (6) to establish the second release position.

9. The system of one of claims 5 to 8, wherein
the receiving part (7) further includes the bore (700) extending from a first end (72) to a second end (73) of the receiving part (7), the rod receiving channel (74) extending from the first end (72), and a seat (75) providing an accommodation space for a head (91) of the bone anchoring element (9) is formed at the second end (72), wherein an inner cap (8) is movably disposed within the accommodation space, which inner cap (8) includes a flexible head receiving portion (82) configured to resiliently clamp the head (91), and
the resilient member (6) is configured to be tensioned by a force in order to achieve a state, when the plunger (5) releases the distal end portion (31), which force is less than a clamping force exerted by the flexible head receiving portion (82) of the inner cap (8).

10. The system of one of claims 1 to 9, wherein
the distal end portion (31) of the collet (3) includes two or more flexible tongues (311, 312), which are configured to be flexibly expanded (E) and/or compressed (C) in a radial direction (R) perpendicular to the longitudinal axis (Z).

11. The system of one of claims 1 to 10, wherein
the engagement feature (34) is a protrusion or latching recess, which is configured to latch into a corresponding latching recess or protrusion, respectively, provided at the inner wall (71, 79) of the receiving part (7).

12. The system of claim 11, wherein two or more of the engagement features (34) are provided.

13. The system according to one of claims 1 to 8, the receiving part (7) further comprising:
a bore (700) extending from a first end (72) to a second end (73) of the receiving part (7) and comprising an inner wall (71),
the rod receiving channel (74) extending from the first end (72) and comprising an inner wall (79),
an opening (701) of the bore (700) provided at the second end (73) and being configured to allow introducing the head (91) into the bore (700), and
a seat (75) formed adjacent the opening (701) and providing an accommodation space for the head (91) of the bone anchoring element (9),
wherein the inner wall (79) of the rod receiving channel or the inner wall (71) of the bore (700) comprises an engagement feature (77) configured to engage a corresponding engagement feature (34) of the device for placing a receiving part onto a head of a bone anchoring element.

14. The system of claim 13, further comprising
an inner cap (8) comprising a flexible head receiving portion (82) configured to resiliently clamp the head (91), the inner cap (8) being movably disposed within the accommodation space.

## Patentansprüche

1. Ein System aus einer Vorrichtung (1) zum Platzieren eines Aufnahmeteils (7) einer Knochenverankerungsvorrichtung auf einem Kopf (91) eines Knochenverankerungselements (9) und dem Aufnahmeteil (7), umfassend:
das Aufnahmeteil (7); und
die Vorrichtung (1), welche ferner umfasst
eine Haltevorrichtung (2),
eine Spannhülse (3) mit einer Längsachse (Z), die bewegbar durch die Haltevorrichtung (2) gehalten wird und eine Röhrenform besitzt, wobei die Spannhülse (3) einen distalen Endabschnitt (31) aufweist, der eingerichtet ist, in einer radialen Richtung (R) senkrecht zu der Längsachse (Z) flexibel expandiert (E) und/oder zusammengedrückt zu werden,
wobei an der Außenkontur des distalen Endabschnitts (31) der Spannhülse (3) ein Eingriffsmerkmal (34) vorgesehen ist, dass eingerichtet ist, in ein Eingriffsmerkmal (77) einer U-förmigen Innenwand (79) einzugreifen, die in einem Stabaufnahmekanal (74) des Aufnahmeteils (7) ausgebildet ist,
einen Kolben (5), der eingerichtet ist, den distalen Endabschnitt (31) der Spannhülse (3) an dem Aufnahmeteil (7) wahlweise zu sperren oder freizugeben, wenn das Eingriffsmerkmal (34) in das in dem Aufnahmeteil (7) ausgebildete Eingriffsmerkmal (77) der Innenwand (79) eingreift, wobei der Kolben (5) eine Stabform besitzt und sich zumindest teilweise durch die Spannhülse (3) hindurch erstreckt.

2. Das System gemäß Anspruch 1, wobei
die Spannhülse (3) eingerichtet ist, in Bezug auf den Kolben (5) in einer Richtung entlang der Längsachse (Z) bewegbar zu sein zwischen wenigstens
einer ersten Sperrposition, in welcher der distale Endabschnitt (31) der Spannhülse (3) durch einen Abschnitt des Kolben (5) daran gehindert wird, zusammengedrückt zu werden, und
einer zweiten Freigabeposition, in welcher der distale Endabschnitt (31) federnd zusammengedrückt werden kann (C).

3. Das System gemäß Anspruch 2, wobei
der Kolben (5) fest mit der Haltevorrichtung (2) gekoppelt ist.

4. Das System gemäß einem der Ansprüche 1-3, wobei
der Kolben (5) mit der Haltevorrichtung (2) über eine Fixiervorrichtung (21) gekoppelt ist, die sich durch eine in der röhrenförmigen Spannhülse (3) erstreckende längliche Öffnung (38) hindurch erstreckt.

5. Das System gemäß einem der Ansprüche 1-4, ferner umfassend:
ein federndes Element (6), welches zwischen der Haltevorrichtung (2) und einem proximalen Endabschnitt (35) an der Spannhöhe (3) vorgesehen ist, um die Spannhülse (3) in einer proximalen Richtung vorzuspannen, um die erste Sperrposition zu herzustellen.

6. Das System gemäß Anspruch 5, wobei
der Kolben (5) einen Spitzenabschnitt (52) besitzt, welcher eingerichtet ist, in der ersten Sperrposition in den distalen Endabschnitt (31) der Spannhülse (3) hineinzutreten, um einem Zusammendrücken (C) desselben vorzubeugen.

7. Das System gemäß Anspruch 6, wobei
der Spitzenabschnitt (52) des Kolbens (5) ferner einen sich verjüngenden oder konischen Abschnitt (55) umfasst, der eingerichtet ist, über dem Vorbeugen des Zusammendrückens hinaus den distalen Endabschnitt (31) der Spannhülse (3) zu expandieren.

8. Das System gemäß einem der Ansprüche 5-7, ferner umfassend:
einen Betätigungsabschnitt (4), welcher mit dem an der Spannhülse (3) vorgesehenen proximalen Abschnitt (35) entfernbar verbunden, fixiert oder integral ausgebildet und eingerichtet ist, es einem Bediener zu erlauben, die Spannhülse (3) in Bezug auf die Haltevorrichtung (2) und den Kolben (5) gegen eine Spannkraft des federnden Elements (6) zu bewegen, um die zweite Freigabeposition herzustellen.

9. Das System gemäß einem der Ansprüche 5-8, wobei
das Aufnahmeteil (7) ferner die Bohrung (700) aufweist, die sich von einem ersten Ende (72) zu einem zweiten Ende (73) des Aufnahmeteils (7) erstreckt, wobei sich der Stabaufnahmekanal (74) vom ersten Ende (72) her erstreckt, und
ein Sitz (75), der einen Aufnahmeraum für einen Kopf (91) des Knochenverankerungselements (9) bereitstellt, an dem zweiten Ende (72) ausgebildet ist, wobei eine innere Kappe (8) bewegbar innerhalb des Aufnahmeraums angeordnet ist, welche innere Kappe (8) einen flexiblen Kopfaufnahmeabschnitt (82) aufweist, der eingerichtet ist, den Kopf (91) federnd zu klemmen, und
das federnde Element (6) eingerichtet ist, durch eine Kraft gespannt zu werden, um einen Zustand zu erreichen, in welchem der Kolben (5) den distalen Endabschnitt (31) freigibt, welche Kraft weniger beträgt als eine Klemmkraft, die vom flexiblen Kopfaufnahmeabschnitt (82) auf die innere Kappe (8) ausgeübt wird.

10. Das System gemäß einem der Ansprüche 1-9, wobei
der distale Endabschnitt (31) der Spannhülse (3) zwei oder mehr flexible Zungen (311, 312) aufweist, welche eingerichtet sind, in einer radialen Richtung (R) senkrecht zu der Längsachse (Z) flexibel expandiert (E) und/oder zusammengedrückt (C) zu werden.

11. Das System gemäß einem der Ansprüche 1-10, wobei
das Eingriffsmerkmal (34) ein Vorsprung oder eine Einrastausnehmung ist, der/die eingerichtet ist, in eine entsprechende Einrastausnehmung oder einen entsprechenden Vorsprung einzurasten, die/der an der Innenwand (71, 79) des Aufnahmeteils (7) vorgesehen ist.

12. Das System gemäß Anspruch 11, wobei zwei oder mehr Eingriffsmerkmale (34) vorgesehen sind.

13. Das System gemäß einem der Ansprüche 1-8, wobei das Aufnahmeteil (7) ferner umfasst:
eine sich von einem ersten Ende (72) zu einem zweiten Ende (73) des Aufnahmeteils (7) erstreckende Bohrung (700), umfassend eine Innenwand (71),
den sich von dem ersten Ende (72) her erstreckenden Stabaufnahmekanal (74), umfassend eine Innenwand (79),
eine Öffnung (701) der Bohrung (700), die an den zweiten Ende (73) vorgesehen und eingerichtet ist, es einem Kopf (91) zu erlauben, in die Bohrung (700) eingeführt zu werden, und
einen Sitz (75) der benachbart zu der Öffnung (701) ausgebildet ist und einen Aufnahmeraum für den Kopf (91) des Knochenverankerungselements (9) bereitstellt,
wobei die Innenwand (79) des Stabaufnahmekanals oder die Innenwand (71) der Bohrung (700) ein Eingriffsmerkmal (77) umfasst, dass eingerichtet ist, in ein entsprechendes Eingriffsmerkmal (34) der Vorrichtung zum Platzieren eines Aufnahmeteils auf einen Kopf eines Knochenverankerungselements einzugreifen.

14. Das System gemäß Anspruch 13, ferner umfassend:
eine innere Kappe (8) umfassend einen flexiblen Kopfaufnahmeabschnitt (82), der eingerichtet ist, den Kopf (91) federnd zu klemmen, wobei die innere Kappe (8) bewegbar innerhalb des Aufnahmeraums angeordnet ist.

## Revendications

1. Système constitué d'un dispositif (1) pour placer une partie de réception (7) d'un dispositif d'ancrage osseux sur une tête (91) d'un élément d'ancrage osseux (9) et de la partie de réception (7), comprenant :
la partie de réception (7) ; et
le dispositif (1), qui comprend en outre
un dispositif de retenue (2),
un collet (3) ayant un axe longitudinal (Z) et étant retenu de manière déplaçable par le dispositif de retenue (2) et ayant une forme tubulaire, le collet (3) comportant une portion d'extrémité distale (31) configurée pour être élargie (E) et/ou comprimée (C) de manière flexible dans une direction radiale (R) perpendiculaire à l'axe longitudinal (Z),
un élément d'engagement (34) étant prévu au niveau d'un contour extérieur de la portion d'extrémité distale (31) du collet (3), configuré pour venir en prise avec un élément d'engagement (77) d'une paroi interne en forme de U (79) formée dans un canal de réception de tige (74) de la partie de réception (7),
un plongeur (5) configuré pour verrouiller et libérer de manière sélective la portion d'extrémité distale (31) du collet (3) au niveau de la partie de réception (7) lorsque l'élément d'engagement (34) s'engage avec l'élément d'engagement (77) de la paroi interne (79) formée dans la partie de réception (7),
le plongeur (5) ayant une forme de tige et s'étendant au moins en partie à travers le collet (3) .

2. Système selon la revendication 1, dans lequel le collet (3) est configuré pour être déplacé par rapport au plongeur (5) dans une direction le long de son axe longitudinal (Z) entre au moins
une première position de verrouillage dans laquelle une portion du plongeur (5) empêche la portion d'extrémité distale (31) du collet (3) d'être comprimée, et
une deuxième position de libération dans laquelle la portion d'extrémité distale (31) peut être comprimée (C) de manière élastique.

3. Système selon la revendication 2, dans lequel le plongeur (5) est accouplé fixement au dispositif de retenue (2).

4. Système selon l'une quelconque des revendications 1 à 3, dans lequel
le plongeur (5) est accouplé au dispositif de retenue (2) par le biais d'un dispositif de fixation (21) s'étendant à travers une ouverture oblongue (38) formée dans le collet de forme tubulaire (3).

5. Système selon l'une quelconque des revendications 1 à 4, comprenant en outre :
un organe élastique (6) qui est accouplé entre le dispositif de retenue (2) et une portion d'extrémité proximale (35) prévue au niveau du collet (3) de manière à pousser le collet (3) dans une direction proximale afin d'établir la première position de verrouillage.

6. Système selon la revendication 5, dans lequel le plongeur (5) présente une portion de pointe (52) qui est configurée, dans la première position de verrouillage, pour pénétrer dans la portion d'extrémité distale (31) du collet (3) de manière à empêcher sa compression (C).

7. Système selon la revendication 6, dans lequel la portion de pointe (52) du plongeur (5) comprend en outre une portion effilée ou conique (55) configurée pour élargir davantage la portion d'extrémité distale (31) du collet (3) en plus d'empêcher sa compression.

8. Système selon l'une des revendications 5 à 7, comprenant en outre
une portion d'actionnement (4) qui est connectée de manière amovible, qui est fixée, ou qui est formée de manière intégrale, avec la portion proximale (35) prévue au niveau du collet (3), configurée pour permettre à un opérateur de déplacer le collet (3) par rapport au dispositif de retenue (2) et un plongeur (5) à l'encontre d'une force de poussée de l'organe élastique (6) pour établir la deuxième position de libération.

9. Système selon l'une des revendications 5 à 8, dans lequel
la partie de réception (7) comporte en outre l'alésage (700) s'étendant depuis une première extrémité (72) jusqu'à une deuxième extrémité (73) de la partie de réception (7), le canal de réception de tige (74) s'étendant depuis la première extrémité (72), et un siège (75) fournissant un espace de réception pour une tête (91) de l'élément d'ancrage osseux (9) est formé au niveau de la deuxième extrémité (72), un capuchon interne (8) étant disposé de manière déplaçable à l'intérieur de l'espace de réception, lequel capuchon interne (8) comporte une portion de réception de tête flexible (82) configurée pour serrer de manière élastique la tête (91), et
l'organe élastique (6) est configuré pour être tendu par une force afin d'atteindre un état, lorsque le plongeur (5) libère la portion d'extrémité distale (31), laquelle force est inférieure à une force de serrage exercée par la portion de réception de tête flexible (82) du capuchon interne (8).

10. Système selon l'une des revendications 1 à 9, dans lequel
la portion d'extrémité distale (31) du collet (3) comporte deux ou plus de deux langues flexibles (311, 312) qui sont configurées pour être élargies (E) et/ou comprimées (C) dans une direction radiale (R) perpendiculaire à l'axe longitudinal (Z).

11. Système selon l'une des revendications 1 à 10, dans lequel
l'élément d'engagement (34) est une saillie ou un renfoncement d'enclenchement qui est configuré pour s'enclencher dans un renfoncement d'enclenchement correspondant ou une saillie, respectivement, prévu(e) au niveau de la paroi interne (71, 79) de la partie de réception (7).

12. Système selon la revendication 11, dans lequel deux ou plus de deux des éléments d'engagement (34) sont prévus.

13. Système selon l'une des revendications 1 à 8, la partie de réception (7) comprenant :
un alésage (700) s'étendant depuis une première extrémité (72) jusqu'à une deuxième extrémité (73) de la partie de réception (7) et comprenant une paroi interne (71),
le canal de réception de tige (74) s'étendant depuis la première extrémité (72) et comprenant une paroi interne (79),
une ouverture (701) de l'alésage (700) prévue au niveau de la deuxième extrémité (73) et configurée pour permettre l'introduction de la tête (91) dans l'alésage (700), et
un siège (75) formé à côté de l'ouverture (701) et fournissant un espace de réception pour la tête (91) de l'élément d'ancrage osseux (9),
la paroi interne (79) du canal de réception de tige ou de la paroi interne (71) de l'alésage (700) comprenant un élément d'engagement (77) configuré pour s'engager avec un élément d'engagement correspondant (34) du dispositif pour placer une partie de réception sur une tête d'un élément d'ancrage osseux.

14. Système selon la revendication 13, comprenant en outre
un capuchon interne (8) comprenant une portion de réception de tête flexible (82) configurée pour serrer de manière élastique la tête (91), le capuchon interne (8) étant disposé de manière déplaçable à l'intérieur de l'espace de réception.
